# EUROPEAN PATENT APPLICATION

(11) **EP 3 858 327 A1**
(43) Date of publication of application: **04.08.2021**
(21) Application number: 19864432.0
(22) Date of filing: 26.09.2019
(51) Int. Cl.: A61K 8/81, A61K 8/58, A61K 8/891, A61Q 19/08

(54) **EXTERNAL PREPARATION COMPOSITION**

(30) Priority: 28.09.2018 WO PCT/JP2018/036563
(71) Applicant: Kao Corporation, Chuo-ku, Tokyo 103-8210 (JP)
(72) Inventor: IIDA, Masayuki, Tokyo 131-8501 (JP)
(74) Representative: Hoffmann Eitle
(86) International application number: PCT/JP2019/038003
(87) International publication number: WO 2020/067360

(57) **Abstract**

The present invention relates to an external preparation composition containing a polymer (A) containing a norbornane structure and hexamethyldisiloxane (B).

## Description

### Field of the Invention

The present invention relates to an external preparation composition.

### Background of the Invention

Aging increases wrinkles on the skin of a human. This is a part of an aging phenomenon of the skin and is influenced by plural factors, such as changes in a skin tissue and effects of exposure to ultraviolet rays.

Techniques have been proposed to make the wrinkles on the skin inconspicuous. However, cosmetic surgery methods required shape control by invading the skin. Although skin care products that improve the wrinkles on the skin in a non-invasive manner are also known, an improvement effect thereof is extremely low, and women with wrinkle problems have not been satisfied.

Then, there is a demand for an external preparation composition capable of controlling the shape of the skin non-invasively and effectively improving the wrinkles.

Now, cosmetics in which a film-forming polymer is blended for the purposes of improving the uniformity of a cosmetic film and the sustainability of the cosmetic are known. PTL 1 relates to a makeup cosmetic with excellent applicability and uniformity of a cosmetic film, in which a predetermined fructooligosaccharide fatty acid ester, a volatile hydrocarbon, and an oil-soluble film-forming resin are blended. PTL 2 relates to a cosmetic containing a film-forming polymer in which a predetermined functional group is introduced into a skeleton of a polycycloolefin polymer, and having good makeup sustainability and feel.

### Citation List

### Patent Literature

PTL 1: JP 2008-247761 A
PTL 2: JP 2012-17317 A

### Summary of the Invention

The present invention relates an external preparation composition containing a polymer (A) containing a norbornane structure and hexamethyldisiloxane (B).

### Detailed Description of the Invention

### [External Preparation composition]

The present invention relates an external preparation composition containing a polymer (A) containing a norbornane structure and hexamethyldisiloxane (B). The foregoing composition is hereinafter also appropriately referred to as "composition of the present invention".

The present inventor has found that by applying an external preparation composition containing the component (A) and the component (B) to a skin, the shape of the skin can be controlled non-invasively to make the wrinkles on the skin inconspicuous.

The term "the wrinkles on the skin" as referred to in this specification means irregularities and streaks that appear on the skin surface. The wrinkles on the skin are liable to appear around the mouth, around the eyes, forehead, neck, and so on. Small streaks are fine wrinkles, and large streaks are large wrinkles. The large wrinkles are caused by a flow of pores on the nasolabial fold, cheeks, and the like. The external preparation composition of the present invention brings about an effect of making any wrinkles inconspicuous, and in particular, it is suitable from the standpoint that even large wrinkles, such as nasolabial fold, can be improved non-invasively.

### <Component (A)>

The composition of the present invention contains a polymer (A) containing a norbornane structure. When the composition containing the component (A) is applied by, for example, applying it the skin in the periphery of wrinkles, and subsequently dried, the film containing the component (A) shrinks, and the skin surface closely adhered to the film also shrinks. By this action, the skin at the wrinkled portion is allowed to smooth out, so that the wrinkles can be made inconspicuous. In addition, in view of the fact that the component (A) is also excellent in flex resistance, a film containing the component (A) hardly causes cracking, and easily shrinks while following the skin shape. Therefore, even on the occasion when the film containing the component (A) shrinks, sufficient adhesion between the film and the skin surface can be obtained.

The norbornane structure as referred to in this specification means a structure represented by the following formula. The component (A) may have a structure represented by the foregoing formula in an arbitrary site in the polymer.

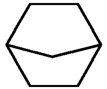

Although the component (A) is not particularly restricted so long as it is a polymer having the aforementioned norbornane structure in a molecule thereof, from the viewpoint of allowing the wrinkles on the skin to smooth out due to the aforementioned mechanisms of action to make them inconspicuous, thereby obtaining a sufficient wrinkle-improving effect (hereinafter also referred to as "skin wrinkle-improving effect"), the component (A) is more preferably a norbornane structure-containing silicone-modified polymer.

Examples of the norbornane structure-containing silicone-modified polymer include a polymer having a repeating unit represented by the following general formula (1) or (2).

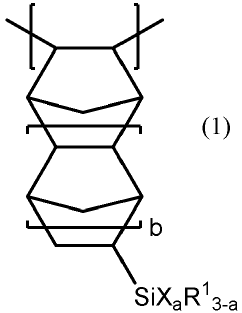

In the formula, R¹'s are each independently an alkyl group having 1 or more and 12 or less carbon atoms; X is a group represented by the following formula (i); a is an integer of 1 or more and 3 or less; and b is an integer of 0 or more and 2 or less.

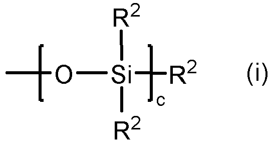

In the formula, R²'s are each independently a hydrocarbon group having 1 or more and 12 or less carbon atoms; and c is an integer of 1 or more and 5 or less.

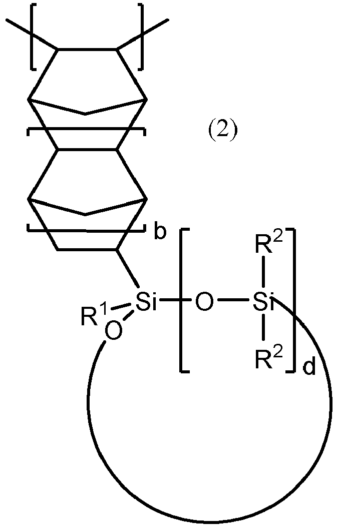

In the formula, R¹, R², and b are the same as those mentioned above; and d is an integer of 2 or more and 5 or less.

In the general formula (1), R¹'s are each independently an alkyl group having 1 or more and 12 or less carbon atoms, and from the viewpoint of the skin wrinkle-improving effect and versatility, R¹ is preferably a methyl group, an ethyl group, a n-propyl group, a butyl group, or a pentyl group, and more preferably a methyl group.

X is a group represented by the aforementioned formula (i), and in the formula (i), R²'s are each independently a hydrocarbon group having 1 or more and 12 or less carbon atoms. From the viewpoint of the skin wrinkle-improving effect and versatility, R² is preferably an alkyl group having 1 or more and 12 or less carbon atoms or an aryl group having 6 or more and 12 or less carbon atoms, more preferably an alkyl group having 1 or more and 12 or less carbon atoms or a phenyl group, still preferably an alkyl group having 1 or more and 3 or less carbon atoms, and yet still more preferably a methyl group. c is an integer of 1 or more and 5 or less, and from the viewpoint of versatility, c is preferably 1. That is, X is preferably a trimethylsiloxy group.
a is an integer of 1 or more and 3 or less, and the foregoing polymer may also be, for example, a polymer in which a repeating unit of a = 2 and a repeating unit of a = 3 are present as a mixture. From the viewpoint of versatility, a is preferably 3. b is an integer of 0 or more and 2 or less, and from the same viewpoint, b is preferably 0, 1, or a combination thereof, and more preferably 0.

In the general formula (2), R¹, R², and b are the same as those mentioned above. d is an integer of 2 or more and 5 or less, and from the viewpoint of versatility, d is preferably 2. From the same viewpoint, the cyclic silicone structure in the general formula (2), it is preferred that R¹ and R² are a methyl group, and d is 2 or 3. That is, the cyclic silicone structure in the general formula (2) is preferably a structure represented by the following formula (4) or (5).

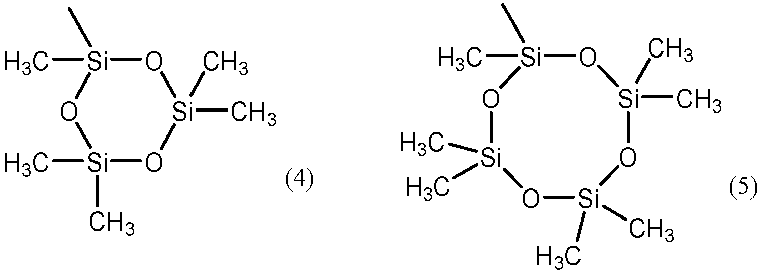

From the viewpoint of the skin winkle-improving effect, a ratio of the repeating unit represented by the general formula (1) or (2) in the norbornane structure-containing silicone-modified polymer is preferably 10% or more, more preferably 30% or more, and still more preferably 50% or more in all of the repeating units in the polymer. In addition, an upper limit thereof is 100%, preferably 95% or less, more preferably 90% or less, and still more preferably 70% or less. A specific range of the number of repeating units represented by the general formula (1) or (2) in the norbornane structure-containing silicone-modified polymer is preferably 10 to 100%, more preferably 10 to 95%, still more preferably 30 to 90%, and yet still more preferably 50 to 70% in the number of all repeating units in the polymer.

The norbornane structure-containing silicone-modified polymer may have a repeating unit represented by the following general formula (3) in addition to the repeating unit represented by the general formula (1) or (2).

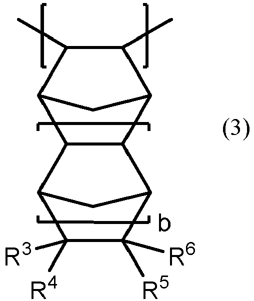

In the formula, R³ to R⁶ are each independently a substituent selected from a hydrogen atom, a halogen atom, and an alkyl group, an alkenyl group, a cycloalkyl group, an aryl group, an alkoxy group, an aryloxy group, and a halogenated hydrocarbon group, each having 1 or more and 10 or less carbon atoms; or an oxetanyl group, an alkoxycarbonyl group, a polyoxyalkylene group, a polyglyceryl group, or an alkoxysilyl group. Two groups selected from R³ to R⁶ may be bonded to each other to form an alicyclic structure, an aromatic ring structure, a carboimide group, or an acid anhydride group. b is the same as that mentioned above.

From the viewpoint of the skin wrinkle-improving effect, a ratio of the repeating unit represented by the general formula (3) in the norbornane structure-containing silicone-modified polymer is preferably 90% or less, more preferably 70% or less, and still more preferably 50% or less in the number of all repeating units in the polymer. In addition, in the case of containing the repeating unit represented by the general formula (3), a ratio thereof is preferably 5% or more, more preferably 10% or more, and still more preferably 30% or more in the number of all repeating units in the polymer. A specific range of the number of repeating units represented by the general formula (3) in the norbornane structure-containing silicone-modified polymer is preferably 5 to 90%, more preferably 10 to 70%, and still more preferably 30 to 50% in the number of all repeating units in the polymer.

The ratio of the repeating units represented by the general formulae (1) to (3) in the norbornane structure-containing silicone-modified polymer can be determined through the ¹H-NMR measurement.

The norbornane structure-containing silicone-modified polymer is preferably a silicone-modified polynorbornene, and more preferably a silicone-modified polynorbornene represented by the following formula (6).

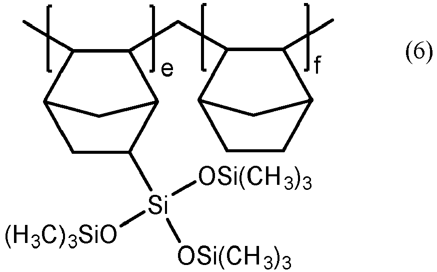

In the formula, e and f are each independently the number of repeating units and are an integer of 1 or more.

From the viewpoint of the skin wrinkle-improving effect, a ratio of e and f in the general formula (6) is preferably e/f = 20/80 to 90/10 (mol/mol), more preferably 30/70 to 80/20 (mol/mol), and still more preferably 50/50 to 70/30 (mol/mol) .

From the viewpoint of making both shrinkability and flex resistance compatible with each other and the viewpoint of the skin wrinkle-improving effect, a number average molecular weight Mn of the norbornane structure-containing silicone-modified polymer is preferably 50,000 or more, more preferably 100,000 or more, and still more preferably 200,000 or more, and it is preferably 2,000,000 or less, more preferably 1,500,000 or less, still more preferably 800,000 or less, and yet still more preferably 600,000 or less. A specific range of the number average molecular weight Mn of the norbornane structure-containing silicone-modified polymer is preferably 50,000 to 2,000,000, more preferably 100,000 to 1,500,000, still more preferably 200,000 to 800,000, and yet still more preferably 200,000 to 600,000.

The number average molecular weight Mn of the polymer can be measured by the gel permeation chromatography (GPC) using polystyrene as a standard substance, and specifically, it can be measured by a method described in the section of Examples.

The component (A) is, for example, obtained by addition polymerization of a cyclic olefin monomer containing a norbornane structure or capable of forming a norbornane structure, using a known method.

For example, if the component (A) is a norbornane structure-containing silicone-modified polymer having a repeating unit represented by the general formula (1) or (2), it can be obtained by addition polymerization of a cyclic olefin monomer represented by the following general formula (1a) or (2a). Furthermore, a cyclic olefin monomer represented by the following general formula (3a) may be copolymerized.

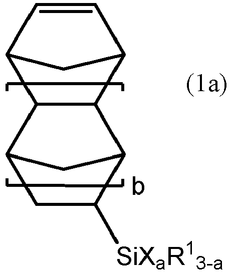

In the formula, R¹'s are each independently an alkyl group having 1 or more and 12 or less carbon atoms; X is a group represented by the following formula (i); a is an integer of 1 or more and 3 or less; and b is an integer of 0 or more and 2 or less.

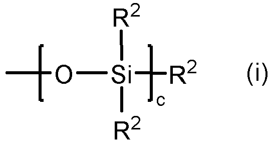

In the formula, R²'s are each independently a hydrocarbon group having 1 or more and 12 or less carbon atoms; and c is an integer of 1 or more and 5 or less.

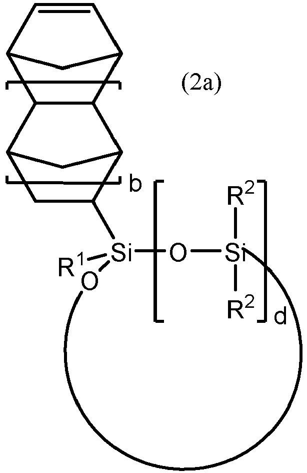

In the formula, R¹, R², and b are the same as those mentioned above; and d is an integer of 2 or more and 5 or less.

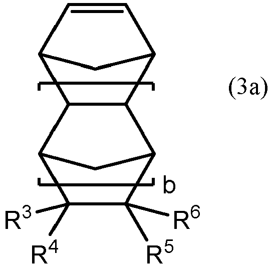

In the formula, R³ to R⁶ are each independently a substituent selected from a hydrogen atom, a halogen atom, and an alkyl group, an alkenyl group, a cycloalkyl group, an aryl group, an alkoxy group, an aryloxy group, and a halogenated hydrocarbon group, each having 1 or more and 10 or less carbon atoms; or an oxetanyl group, an alkoxycarbonyl group, a polyoxyalkylene group, a polyglyceryl group, or an alkoxysilyl group. Two groups selected from R³ to R⁶ may be bonded to each other to form an alicyclic structure, an aromatic ring structure, a carboimide group, or an acid anhydride group. b is the same as that mentioned above.

In the case where the cyclic olefin monomer represented by the general formula (3a) is copolymerized, from the viewpoint of the skin wrinkle-improving effect, the amount of use thereof is preferably 90 mol% or less, more preferably 70 mol% or less, and still more preferably 50 mol% or less, and it is preferably 5 mol% or more, more preferably 10 mol% or more, and still more preferably 30 mol% or more, based on 100 mol% of all monomers to be used for the polymerization.

The silicone-modified polynorbornene represented by the formula (6) can be obtained by addition polymerization of tris(trimethylsiloxy)silylnorbornene represented by the following formula (6a) and norbornene.

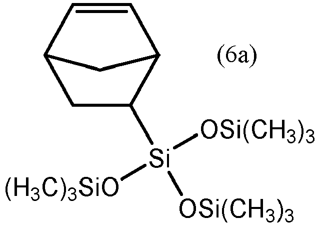

From the viewpoint of the skin wrinkle-improving effect, a copolymerization ratio of tris(trimethylsiloxy)silylnorbornene to norbornene is preferably 20/80 to 90/10 (mol/mol), more preferably 30/70 to 80/20 (mol/mol), and still more preferably 50/50 to 70/30 (mol/mol).

Although the repeating units represented in the general formulae (1) to (3) and the formula (6) all represent a 2,3-addition structural unit of the cyclic olefin monomer that is the raw material monomer, one which becomes a 2,7-addition structural unit due to addition polymerization of the foregoing cyclic olefin monomer may be included.

As the norbornane structure-containing silicone-modified polymer, a norbornene/tris(trimethylsiloxy)silylnorbornene copolymer is preferred, and this is a compound expressed by the INCI Name (International Cosmetic Ingredient Dictionary and Handbook, 16th Edition, Volume 2, 2016, p.2274): NORBORNENE/TRIS(TRIMETHYLSILOXY)SILYLNORBORNENE COPOLYMER.

Examples of a commercially available product of the norbornane structure-containing silicone-modified polymer include "NBN-30-ID" (an isododecane solution of a norbornene/tris(trimethylsiloxy)silylnorbornene copolymer), manufactured by Shin-Etsu Chemical Co., Ltd.

From the viewpoint of the skin wrinkle-improving effect, the content of the component (A) in the composition of the present invention is preferably 0.1% by mass or more, more preferably 0.5% by mass or more, still more preferably 1% by mass or more, and yet still more preferably 5% by mass or more. In addition, from the viewpoint of applicability of the composition, flexibility of the prescription, and not giving an uncomfortable feeling to the skin after drying, the content of the component (A) in the composition is preferably 30% by mass or less, more preferably 25% by mass or less, still more preferably 20% by mass or less, and yet still more preferably 15% by mass or less. A specific range of the content of the component (A) in the composition is preferably 0.1 to 30% by mass, more preferably 0.5 to 25% by mass, still more preferably 1 to 20% by mass, yet still more preferably 1 to 15% by mass, and even yet still more preferably 5 to 15% by mass.

### <Component (B)>

From the viewpoint of more enhancing the skin wrinkle-improving effect, the composition of the present invention contains hexamethyldisiloxane as a component (B). In view of the fact that the hexamethyldisiloxane is high in volatility, when it is contained, in view of the fact that the drying speed of the composition containing the component (A) is increased, the shrinkage of the component (A) in the drying process is accelerated, and the effect of allowing the wrinkles on the skin to smooth out due to the aforementioned mechanisms of action to make them inconspicuous is more improved.

The content of the component (B) in the composition of the present invention is preferably 1% by mass or more, more preferably 5% by mass or more, still more preferably 10% by mass or more, yet still more preferably 20% by mass or more, even yet still more preferably 25% by mass or more, even still more preferably 35% by mass or more, and even still more further preferably 45% by mass or more from the viewpoint of more enhancing the skin wrinkle-improving effect, and it is preferably 99.9% by mass or less, more preferably 99.5% by mass or less, still more preferably 99% by mass or less, yet still more preferably 97% by mass or less, and even yet still more preferably 95% by mass or less from the viewpoint of enhancing the skin wrinkle-improving effect and the viewpoint of flexibility of the prescription. A specific range of the component (B) in the composition is preferably 1 to 99.9% by mass, more preferably 5 to 99.9% by mass, still more preferably 10 to 99.5% by mass, yet still more preferably 20 to 99.5% by mass, even yet still more preferably 25 to 99.5% by mass, even still more preferably 35 to 99.5% by mass, even still more further preferably 35 to 99% by mass, even yet still more further preferably 35 to 97% by mass, and more even yet still more further preferably 45 to 95% by mass.

The composition of the present invention may contain an oil other than the component (B). From the viewpoint of improving the drying speed of the composition, as the oil other than the component (B), at least one selected from the group consisting of a hydrocarbon oil and a silicone oil is preferred; at least one selected from the group consisting of isododecane, methyl trimethicone, decamethyl cyclopentasiloxane, and a dimethylpolysiloxane having a kinematic viscosity at 25°C of 2 cSt or less is preferred; at least one selected from the group consisting of isododecane and decamethyl cyclopentasiloxane is more preferred; and isododecane is still more preferred. The aforementioned kinematic viscosity can be, for example, measured using an Ubbelohde viscometer.

However, from the viewpoint of obtaining the effects of the present invention, the content of the oil other than the component (B) in the composition of the present invention is preferably 80% by mass or less, more preferably 70% by mass or less, still more preferably 50% by mass or less, yet still more preferably 40% by mass or less, even yet still more preferably 30% by mass or less, even still more preferably 20% by mass or less, even still more further preferably 10% by mass or less, and even yet still more further preferably 5% by mass or less, and an lower limit thereof is 0% by mass.

### <Other Components>

The external preparation composition of the present invention may appropriately contain, in addition to the aforementioned components, other components which are typically blended in an external preparation composition, such as a cosmetic, within a range where the object of the present invention is not impaired. Examples of the foregoing components include a surfactant, a water-soluble polymer, an antioxidant, a UV absorber, a vitamin, a preservative, a pH modifier, a fragrance, a plant extract, a moisturizer, a coloring agent, a cooling sensation agent, an antiperspirant, a disinfectant, and a skin activator.

The form of the external preparation composition of the present invention may be any form that can be applied to the skin, and examples thereof include a solution, an emulsion, a cream, a lotion, a gel, a pack, a sheet, and a foam.

As for the external preparation composition of the present invention, from the viewpoint of applying it to the skin by, for example, application and subsequently drying the composition, to shrink the component (A), thereby bringing about the skin wrinkle-improving effect, the foregoing composition is preferably liquid at the time of applying to the skin, and more preferably liquid at 25°C.

As for the external preparation composition of the present invention, from the viewpoint of accelerating shrinkage of the component (A) in a step of applying to the skin by, for example, application and subsequently drying the composition, to enhance the skin wrinkle-improving effect, the content of water is preferably low. The content of water in the composition is preferably 15% by mass or less, more preferably 10% by mass or less, still more preferably 5% by mass or less, yet still more preferably 2% by mass or less, even yet still more preferably 1% by mass or less, even still more preferably 0.5% by mass or less, and even still more further preferably 0.1% by mass or less.

The external preparation composition of the present invention can be used as a composition for improving wrinkles on the skin, and specifically, it can be applied by, for example, applying it to the periphery of wrinkles on the skin. According to this, the shape of the skin can be controlled non-invasively by the aforementioned mechanisms of action, to make the wrinkles on the skin inconspicuous.

In accordance with the composition of the present invention, it is also possible to provide a method for improving wrinkles on the skin by applying the composition of the present invention to the skin.

It is preferred that the foregoing method includes a step of applying the composition of the present invention to the periphery of wrinkles on the skin and a step of subsequently drying the composition. When these steps are carried out to pull and allow the skin in the periphery of wrinkles on the skin to smooth out due to shrinkage of the film containing the component (A) that is contained in the composition of the present invention, the shape of the skin can be controlled non-invasively. That is, the method for improving wrinkles on the skin in this specification is a method in which the wrinkles on the skin are physically pulled due to the aforementioned mechanisms of action, to flatten the irregular shape on the skin surface, and the foregoing method is different from, for example, a method of applying an external preparation composition to the skin, thereby filling the irregularities on the skin surface, or optically making the wrinkles inconspicuous.

The "periphery of wrinkles on the skin" in this specification is distinguished from the inside of the wrinkle, that is, the concave part on the skin surface or the concave part of the streak. In the present invention, by applying the composition of the present invention to the skin in the periphery of wrinkles, on the occasion of drying the composition, the skin surface in the periphery of wrinkles shrinks along with the shrinkage of the film containing the component (A), and the skin in the wrinkled is pulled, whereby the wrinkles are made inconspicuous. However, so long as the effects of the present invention are obtained, the composition of the present invention is not prevented from application to not only the periphery of wrinkles but also the inside of the wrinkles.

From the viewpoint of obtaining the aforementioned effects, it is preferred that the area to which the composition is applied includes at least a part of an area intersecting with the direction of the wrinkle in the periphery of wrinkles. The direction of the wrinkle refers to the direction in which the streak runs. This is because by applying the composition to an area intersecting with the direction of the wrinkle, the wrinkled portion is allowed to smooth out, whereby the wrinkles become inconspicuous.

The area intersecting with the direction of the wrinkle is preferably an area along the direction of the wrinkle, and the foregoing area may be either one side or both sides of the wrinkle. In particular, it is more preferred to apply the composition to the both sides sandwiching the wrinkle. This is because by pulling the skin in the winkled portion from the both sides of the wrinkle, the wrinkle can be made more inconspicuous.

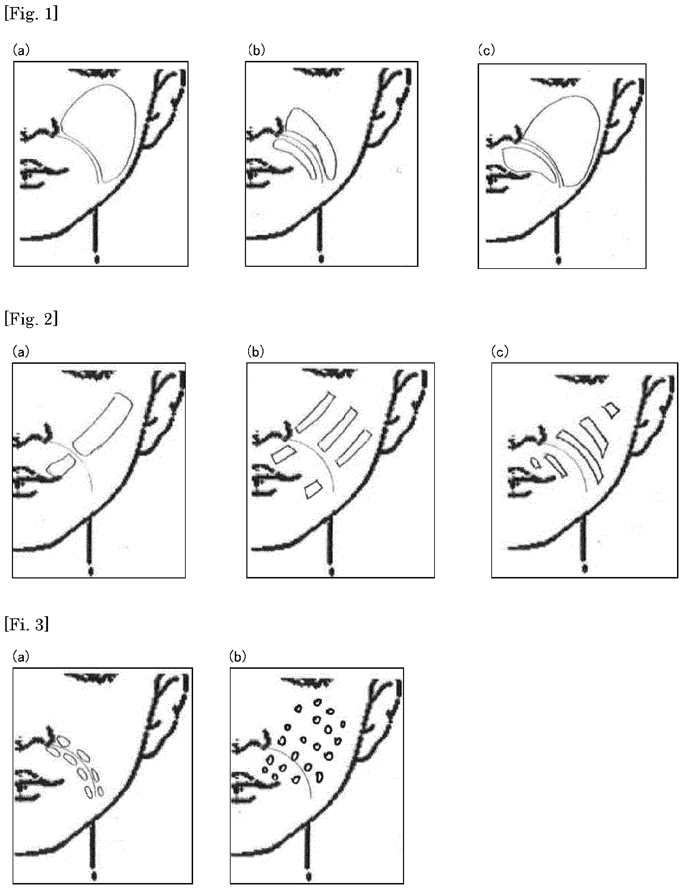

Examples of a form in which the composition is applied to an area along the direction of the wrinkle include a form in which the composition is applied in a planar, band-like, linear, or dot-like shape to the area, and specifically, those illustrated in Figs. 1 to 3 as mentioned later can be exemplified.

In the case where the direction of the wrinkle is not parallel to the direction of gravity, for example, as in a nasolabial fold, it is preferred that the area to which the composition of the present invention is applied (the area intersecting with the direction of the wrinkle) includes an area at the upper side of the wrinkle. The "direction of gravity" as referred to herein means a direction in which the gravity acts in the case where a person stands upright, and the "upper side" means a direction opposite to the direction of gravity. By applying the composition to at least the area at the upper side of the wrinkle, the skin at the wrinkled portion is pulled upward, thereby enabling the wrinkle to be made more inconspicuous. From the same viewpoint as mentioned above, in the case where the direction of the wrinkle is not parallel to the direction of gravity, the area to which the composition of the present invention is applied is an area at the upper and lower sides sandwiching the wrinkle, and it is more preferred that the area at the upper side of the wrinkle is a range wider than the area at the lower side of the wrinkle.

One example of the form in which the composition of the present invention is applied to the periphery of wrinkles on the skin is illustrated in Figs. 1 to 3.

Fig. 1 shows a form in which the composition is applied in a planar or band-like shape to the skin in the periphery of the wrinkle (nasolabial fold), in which Fig. 1(a) shows the state that the composition is applied to the area at the upper side of the wrinkle; and Figs. 1(b) and 1(c) each show the state that the composition is applied to the areas at the upper side and the lower side sandwiching the wrinkle. From the viewpoint of making the wrinkles more inconspicuous, it is more preferred that not only the area to which the composition is applied is the areas at the upper side and the lower side sandwiching the wrinkle, but also the area at the upper side of the wrinkle is wider than the area at the lower side of the wrinkle, as shown in Fig. 1(c).

Figs. 2(a), 2(b), and 2(c) each show the state that the composition is applied in a linear shape to the skin in the periphery of the wrinkle (nasolabial fold); and Figs. 3(a) and 3(b) each show the state that the composition is applied in a dot-like shape to the skin in the periphery of the wrinkle (nasolabial fold). As shown in Figs. 2 and 3, even in the case where the composition is applied in a linear or dot-like shape, from the viewpoint of evenly pulling the skin from the both sides of the wrinkle, it is preferred to apply the composition to the areas at the upper side and the lower side sandwiching the wrinkle.

In the case of applying the composition of the present invention to the skin, though the amount of use thereof is not particularly restricted so long as the effect of making the wrinkles inconspicuous may be developed, it is, for example, an amount of 0.01 g or more and 10 g or less.

It is preferred that the method of applying the composition of the present invention to the skin includes a step of applying the composition, for example, to the periphery of wrinkles on the skin; and a step of subsequently drying the composition to shrink the polymer. Due to the drying, the component (A) in the composition applied to the skin or the like shrinks, and the skin in the periphery of wrinkles is pulled and allowed to smooth out due to this shrinkage action, thereby bringing about an effect of flattening the irregular shape on the skin surface, in its turn, making the wrinkles inconspicuous.

After drying, the composition of the present invention may be removed, as the need arises. Alternatively, after applying the composition to the skin and subsequently drying it, the makeup may be applied over it without removing the composition.

Regarding the aforementioned embodiments, the present invention further discloses the following embodiments.
<1> An external preparation composition containing a polymer (A) containing a norbornane structure and hexamethyldisiloxane (B).
<2> The external preparation composition as set forth in <1>, wherein the content of the polymer (A) containing a norbornane structure is 0.1% by mass or more and 30% by mass or less, and the content of the hexamethyldisiloxane (B) is 1% by mass or more and 99.9% by mass or less.
<3> The external preparation composition as set forth in <1> or <2>, wherein the polymer (A) containing a norbornane structure is a silicone-modified polynorbornene represented by the following formula (6): 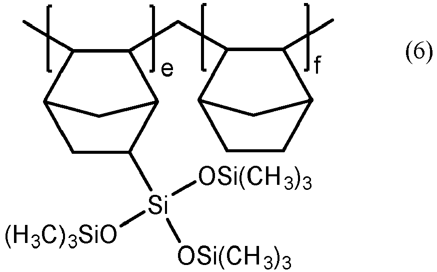 wherein,
   e and f are each independently the number of repeating units and are an integer of 1 or more.
<4> The external preparation composition as set forth in <3>, wherein a ratio of e and f in the formula (6) is e/f = 20/80 to 90/10 (mol/mol).
<5> The external preparation composition as set forth in any one of <1> to <4>, wherein the content of the component (A) is 1% by mass or more and 20% by mass or less.
<6> The external preparation composition as set forth in any one of <1> to <5>, wherein the content of the component (B) is 5% by mass or more and 99.5% by mass or less.
<7> The external preparation composition as set forth in any one of <1> to <6>, wherein the content of the component (B) is 20% by mass or more and 99.5% by mass or less.
<8> The external preparation composition as set forth in any one of <1> to <7>, wherein the content of the component (B) is 35% by mass or more and 99% by mass or less.
<9> The external preparation composition as set forth in <1> or <2>, wherein the content of the component (A) is 1% by mass or more and 20% by mass or less, and the content of the component (B) is 5% by mass or more and 99.5% by mass or less.
<10> The external preparation composition as set forth in <1> or <2>, wherein the content of the component (A) is 1% by mass or more and 20% by mass or less, and the content of the component (B) is 20% by mass or more and 99.5% by mass or less.
<11> The external preparation composition as set forth in <1> or <2>, wherein the content of the component (A) is 1% by mass or more and 20% by mass or less, and the content of the component (B) is 35% by mass or more and 99% by mass or less.
<12> The external preparation composition as set forth in <1> or <2>, wherein the component (A) is a silicone-modified polynorbornene represented by the following formula (6), and the content thereof is 1% by mass or more and 20% by mass or less; and the content of the component (B) is 5% by mass or more and 99.5% by mass or less: 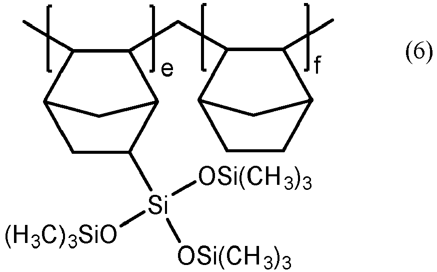 wherein,
   e and f are each independently the number of repeating units and are an integer of 1 or more.
<13> The external preparation composition as set forth in <1> or <2>, wherein the component (A) is a silicone-modified polynorbornene represented by the following formula (6), and the content thereof is 1% by mass or more and 20% by mass or less; and the content of the component (B) is 20% by mass or more and 99.5% by mass or less: 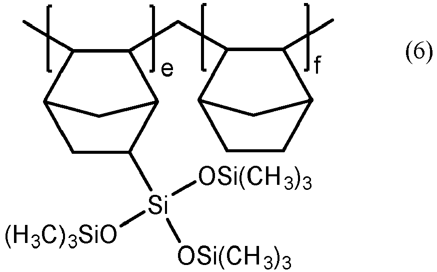 wherein,
   e and f are each independently the number of repeating units and are an integer of 1 or more.
<14> The external preparation composition as set forth in <1> or <2>, wherein the component (A) is a silicone-modified polynorbornene represented by the following formula (6), and the content thereof is 1% by mass or more and 20% by mass or less; and the content of the component (B) is 35% by mass or more and 99% by mass or less: 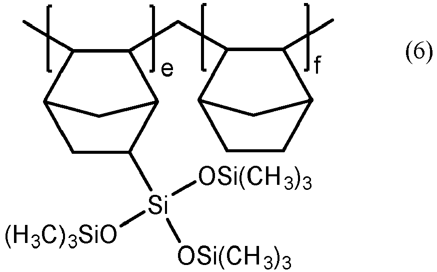 wherein,
   e and f are each independently the number of repeating units and are an integer of 1 or more.
<15> The external preparation composition as set forth in any one of <12> to <14>, wherein a ratio of e and f in the formula (6) is e/f = 20/80 to 90/10 (mol/mol).
<16> The external preparation composition as set forth in any one of <1> to <15>, which is a composition for improving wrinkles on the skin.
<17> The external preparation composition as set forth in any one of <1> to <16>, wherein the content of water is 5% by mass or less.
<18> A method for improving wrinkles on the skin, including applying the external preparation composition as set forth in any one of <1> to <17> to the periphery of wrinkles on the skin.
<19> A method for improving wrinkles on the skin, including a step of applying the external preparation composition as set forth in any one of <1> to <17> to the periphery of wrinkles on the skin; and a step of subsequently drying the composition.
<20> The method as set forth in <18> or <19>, wherein in the periphery of wrinkles, an area to which the external preparation composition is applied includes at least a part of an area intersecting with the direction of the wrinkle.
<21> The method as set forth in <20>, wherein the area is an area along the direction of the wrinkle.
<22> The method as set forth in <20> or <21>, wherein the area is the both sides sandwiching the wrinkle.
<23> The method as set forth in any one of <20> to <22>, wherein the external preparation composition is applied in a planar, band-like, linear, or dot-like shape to the area.
<24> The method as set forth in any one of <18> to <23>, wherein the method for improving wrinkles is a method in which the skin in the periphery of wrinkles is pulled and allowed to smooth out due to a shrinkage action of the component (A), thereby controlling the shape of the skin non-invasively.
<25> Use of the external preparation composition as set forth in any one of <1> to <17> as a composition for improving wrinkles on the skin.

### Examples

The present invention is hereunder described by reference to Examples, but it should be construed that the present invention is not limited to the scope of the Examples. In the present Examples, various measurements and evaluations were performed by the following methods.

### (Number Average Molecular Weight Mn)

The number average molecular weight of the polymer was measured under the following condition by the gel permeation chromatography (GPC) using polystyrene as a standard substance.
Measuring device: HLC-8320GPC (manufactured by Tosoh Corporation)
Column: K-806L (made of Shodex), two columns in series
Detector: RI
Eluent: 1 mmol/L-FARMIN DM20 (manufactured by Kao Corporation)/CHCl₃
Flow rate: 1.0 mL/min
Column temperature: 40°C

### (Wrinkle-Improving Effect)

In the form of Fig. 1 (c), 0.2 g of a composition of each of the Examples was applied with a finger so as to sandwich a nasolabial fold at the mouth of the half face of the subject. After drying at room temperature (25°C) for 10 minutes, an expert evaluator evaluated the wrinkle-improving effect according to the following five-grade criteria.
5: The wrinkles became considerably thin.
4: The wrinkles became thin.
3: The wrinkles became slightly thin.
2: The wrinkles remained and did not change much.
1: No change.

### Examples 1 to 9 and Comparative Examples 1 to 2 (Production and Evaluation of External Preparation Composition)

All of the components shown in Table 1 were blended and uniformly mixed using a Disper, to prepare external preparation compositions shown in Table 1. Using each of the obtained external preparation compositions, the evaluation of the skin wrinkle-improving effect was carried out by the aforementioned method. The results are shown in Table 1. The blending amounts described in Table 1 are amounts (% by mass) of active ingredients of the respective components.

**Table 1**

| Component (% by mass) | | Example | | | | | | | | | Comparative Example | |
|---|---|---|---|---|---|---|---|---|---|---|---|---|
| | | 1 | 2 | 3 | 4 | 5 | 6 | 7 | 8 | 9 | 1 | 2 |
| Component (A) | (Norbornene/tris(trimethylsiloxy)-silylnorobornene) copolymer *1 | 10.0 | 10.0 | 1.0 | 5.0 | 15.0 | 10.0 | 10.0 | 10.0 | 13.0 | 10.0 | |
| Polymer other than component (A) | Acrylic silicone *2 | | | | | | | | | | | 10.0 |
| Component (B) | Hexamethyldisiloxane *3 | 90.0 | 66.7 | 99.0 | 95.0 | 85.0 | 10.0 | 25.0 | 50.0 | 28.8 | | 90.0 |
| Volatile oil other than component (B) | Isododecane | | 23.3 | | | | | | | 58.2 | | |
| | Decamethyl cyclopentasiloxane *4 | | | | | | 80.0 | 65.0 | 40.0 | | 90.0 | |
| Total | | 100.0 | 100.0 | 100.0 | 100.0 | 100.0 | 100.0 | 100.0 | 100.0 | 100.0 | 100.0 | 100.0 |
| Evaluation | Wrinkle-improving effect | 5 | 5 | 5 | 5 | 5 | 3 | 4 | 5 | 4 | 1 | 1 |

| | | | | | | | | | | | | |
|---|---|---|---|---|---|---|---|---|---|---|---|---|
| The blending components of Table 1 are mentioned below. *1: "NBN-30-ID", manufactured by Shin-Etsu Chemical Co., Ltd. (e/f = 60/40 mol/mol in the following formula (6), an isododecane solution of a norbornene/tris(trimethylsiloxy)silylnorbornene) copolymer of Mn = 360,000, active ingredient concentration: 30% by mass) was dried under reduced pressure at 50°C for 12 hours, and the resulting solid was used. 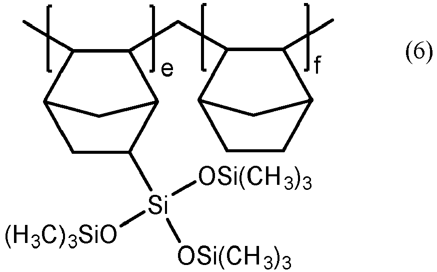 *2: "KP-550", manufactured by Shin-Etsu Chemical Co., Ltd. (an isododecane solution of a graft copolymer composed of an acrylic polymer and a dimethylpolysiloxane, active ingredient concentration: 40% by mass) was dried under reduced pressure at 50°C for 12 hours, and the resulting solid was used. *3: "KF-96L-0.65cs", manufactured by Shin-Etsu Chemical Co., Ltd., hexamethyldisiloxane *4: "KF-995", manufactured by Shin-Etsu Chemical Co., Ltd., decamethyl cyclopentasiloxane | | | | | | | | | | | | |

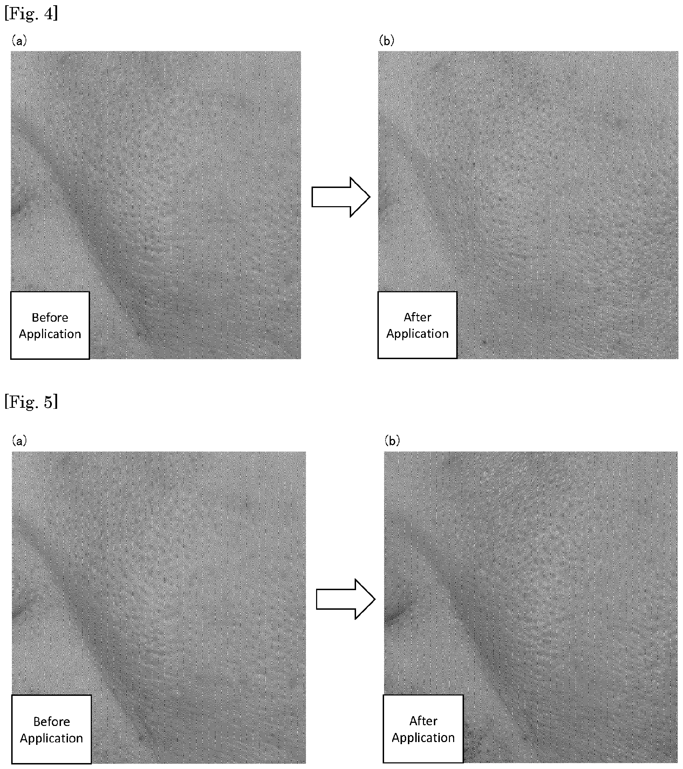

With respect to the wrinkle-improving effect, the results of Example 1 are shown in Fig. 4, and the results of Comparative Example 2 are shown in Fig. 5. Fig. 4(a) and Fig. 5(a) are each a photograph in the periphery of the nasolabial fold before application of the external preparation composition, and Fig. 4(b) and Fig. 5(b) are each a photograph in the periphery of the nasolabial fold after application of the external preparation composition. As shown in Fig. 4, in the case of applying the external preparation composition of Example 1, the nasolabial fold and the fine wrinkles of the periphery thereof became inconspicuous, whereas in Comparative Example 2 shown in Fig. 5, the improving effect was not perceived.

### Industrial Applicability

In accordance with the external preparation composition of the present invention, the shape of the skin can be controlled non-invasively to make the wrinkles on the skin inconspicuous.

### Brief Description of the Drawings

Fig. 1 is an example of a form in which the composition is applied in a planar or band-like shape to the skin in the periphery of the wrinkle (nasolabial fold).
Fig. 2 is an example of a form in which the composition is applied in a linear shape to the skin in the periphery of the wrinkle (nasolabial fold).
Fig. 3 is an example of a form in which the composition is applied in a dot-like shape to the skin in the periphery of the wrinkle (nasolabial fold).
Fig. 4 is a photograph showing the wrinkle-improving effect of Example 1.
Fig. 5 is a photograph showing the wrinkle-improving effect of Comparative Example 2.

## Claims

1. An external preparation composition comprising a polymer (A) containing a norbornane structure and hexamethyldisiloxane (B).

2. The external preparation composition according to claim 1, wherein the component (A) is a norbornane structure-containing silicone-modified polymer.

3. The external preparation composition according to claim 2, wherein the norbornane structure-containing silicone-modified polymer is a polymer having a repeating unit represented by the following general formula (1) or (2): 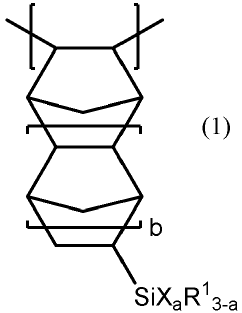 wherein,
R¹'s are each independently an alkyl group having 1 or more and 12 or less carbon atoms; X is a group represented by the following formula (i); a is an integer of 1 or more and 3 or less; and b is an integer of 0 or more and 2 or less, 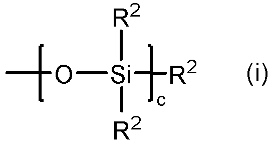 wherein,
R²'s are each independently a hydrocarbon group having 1 or more and 12 or less carbon atoms; and c is an integer of 1 or more and 5 or less, and 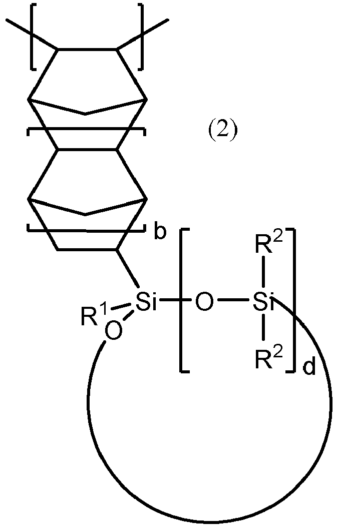 wherein,
R¹, R², and b are the same as those mentioned above; and d is an integer of 2 or more and 5 or less.

4. The external preparation composition according to claim 3, wherein the polymer is a silicone-modified polynorbornene represented by the following formula (6): 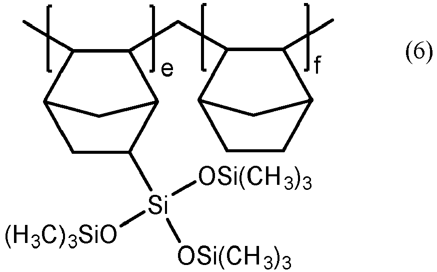 wherein,
e and f are each independently the number of repeating units and are an integer of 1 or more.

5. The external preparation composition according to claim 4, wherein a ratio of e and f in the formula (6) is e/f = 20/80 to 90/10 (mol/mol).

6. The external preparation composition according to any one of claims 1 to 5, wherein the content of the component (A) is 0.1% by mass or more and 30% by mass or less.

7. The external preparation composition according to any one of claims 1 to 6, wherein the content of the component (B) is 1% by mass or more and 99.9% by mass or less.

8. The external preparation composition according to any one of claims 1 to 7, which is a composition for improving wrinkles on the skin.

9. Use of the external preparation composition according to any one of claims 1 to 7 as a composition for improving wrinkles on the skin.
